Europäisches Patentamt

European Patent Office

Office européen des brevets

(11) Veröffentlichungsnummer: **0 523 089 B1**

## EUROPÄISCHE PATENTSCHRIFT

(45) Veröffentlichungstag der Patentschrift: **02.11.94**

(21) Anmeldenummer: **91906666.2**

(22) Anmeldetag: **25.03.91**

(86) Internationale Anmeldenummer:
**PCT/EP91/00566**

(87) Internationale Veröffentlichungsnummer:
**WO 91/15441 (17.10.91 91/24)**

(51) Int. Cl.5: **C07B 41/04**, C07C 41/03,
C07C 67/26, C07C 43/11,
C07C 43/196, C07C 69/22,
C07C 217/08

(54) **VERWENDUNG VON HYDROPHOBIERTEN HYDROTALCITEN ALS KATALYSATOREN FÜR DIE ETHOXYLIERUNG BZW. PROPOXYLIERUNG.**

(30) Priorität: **02.04.90 DE 4010606**

(43) Veröffentlichungstag der Anmeldung:
**20.01.93 Patentblatt 93/03**

(45) Bekanntmachung des Hinweises auf die Patenterteilung:
**02.11.94 Patentblatt 94/44**

(84) Benannte Vertragsstaaten:
**BE DE ES FR GB IT SE**

(56) Entgegenhaltungen:
EP-A- 0 085 167
EP-A- 0 339 426
DE-A- 3 833 076

(73) Patentinhaber: **Henkel Kommanditgesellschaft auf Aktien**

**D-40191 Düsseldorf (DE)**

(72) Erfinder: **RATHS, Hans-Christian, Dr.**
**Hügelstrasse 32**
**D-4000 Düsseldorf 13 (DE)**
Erfinder: **BREUER, Wolfgang, Dr.**
**Karolingerstrasse 22**
**D-4000 Düsseldorf 1 (DE)**
Erfinder: **FRIEDRICH, Klaus, Dr.**
**Marconistrasse 13**
**D-4000 Düsseldorf 13 (DE)**
Erfinder: **HERRMANN, Klaus**
**Köpenicher Strasse 33**
**D-4019 Monheim (DE)**

EP 0 523 089 B1

**Beschreibung**

Die Erfindung betrifft die Verwendung von hydrophobierten Hydrotalciten der allgemeinen Formel I

$$Mg_xAl(OH)_y(CO_3)_m(A)_n . z H_2O \qquad (I)$$

in der
A das Dianion einer aliphatischen Dicarbonsäure mit 4 bis 44 Kohlenstoffatomen oder zwei Anionen von aliphatischen Monocarbonsäuren mit 2 bis 34 Kohlenstoffatomen bedeutet und die Bedingungen

$1 < x < 5$,
$y \geq 2x + 2$
$[y + 2(m + n)] = 2x + 3$,
$m + n \leq 0,5$
$m \geq 0$,
$n > 0$ und
$0 < z < 10$

gelten,
als Katalysatoren für die Ethoxylierung bzw. Propoxylierung von Verbindungen mit aktiven H-Atomen sowie von Fettsäureestern, welche aus der von Estern von gegebenenfalls hydroxysubstituierten Fettsäuren mit 2 bis 22 Kohlenstoffatomen mit Monoalkanolen mit 1 bis 22 Kohlenstoffatomen sowie von Partialestern und Vollestern von gegebenenfalls hydroxy-substituierten Fettsäuren mit 2 bis 22 Kohlenstoffatomen mit Polyolen mit 2 bis 12 Kohlenstoffatomen und 2 bis 6 Hydroxylgruppen gebildeten Gruppe ausgewählt sind.

Hydrotalcit ist ein natürliches Mineral mit der Idealformel $Mg_6Al_2(OH)_{16}CO_3.4H_2O$, dessen Struktur von derjenigen des Brucits ($Mg(OH)_2$) abgeleitet ist. Brucit kristallisiert in einer Schichtstruktur mit den Metallionen in Oktaederlücken zwischen zwei Schichten aus dichtgepackten Hydroxylionen, wobei nur jede zweite Schicht der Oktaederlücken besetzt ist. Im Hydrotalcit sind einige Magnesiumionen durch Aluminiumionen ersetzt, wodurch das Schichtpaket eine positive Ladung erhält. Diese wird durch die Anionen ausgeglichen, die sich zusammen mit zeolithischen Kristallwasser in den Zwischenschichten befinden. Der Schichtaufbau wird in dem Röntgenpulverdiagramm deutlich (ASTM-Karte Nr.14-191), das zur Charakterisierung herangezogen werden kann.

Es sind auch synthetisch hergestellte Hydrotalcite bekannt, die z.B. in den DE-C 1 592 126, DE-A 3 346 943, DE-A 3 306 822 und EP-A 0 207 811 beschrieben sind.

In natürlichen und synthetischen Produkten kann das $Mg^{2+}:Al^{3+}$-Verhältnis zwischen etwa 1 und 5 variieren. Auch das Verhältnis von $OH^-:CO_3^{2-}$ kann schwanken. Natürliche und synthetische Hydrotalcite können durch die allgemeine Formel II

$$Mg_aAl(OH)_b(CO_3)_c . d H_2O \qquad (II)$$

näherungsweise beschrieben werden, wobei die Bedingungen $1 < a < 5$, $b > c$, $(b + 2c) = 2a + 3$ und $0 < d < 10$ gelten. Unterschiede in der Zusammensetzung der Hydrotalcite, insbesondere bezüglich des Wassergehaltes, führen zu Linienverschiebungen im Röntgenbeugungsdiagramm.

Natürliche oder synthetische Hydrotalcite geben beim Erhitzen bzw. Calcinieren kontinuierlich Wasser ab. Die Entwässerung ist bei 200°C vollständig, wobei durch Röntgenbeugung nachgewiesen werden konnte, daß die Struktur des Hydrotalcits noch erhalten geblieben ist. Die weitere Temperaturerhöhung führt unter Abspaltung von Hydroxylgruppen (als Wasser) und von Kohlendioxid zum Abbau der Struktur. Natürliche und nach verschiedenen Verfahren, z.B. gemäß den obigen Veröffentlichungen, künstlich hergestellte Hydrotalcite zeigen bei der Calcinierung ein generell ähnliches Verhalten.

Calcinierte Hydrotalcite sind bereits als Ethoxylierungs- und Propoxylierungskatalysatoren mit ausgezeichneten Ergebnissen eingesetzt worden, vgl. DE-A 38 43 713. Sie weisen lediglich den Nachteil auf, daß sie aus den natürlichen und synthetischen Hydrotalciten durch mehrstündiges Erhitzen auf Temperaturen von z.B. 400 bis 600°C, in eine für katalytische Zwecke geeignete, calcinierte Form überführt werden müssen.

Hydrophobierte Hydrotalcite, bei denen die Carbonationen vollständig oder teilweise durch Anionen von Säuren, z.B. auch Fettsäuren, ersetzt wurden, wurden bereits als Stabilisatoren für thermoplastische Harze eingesetzt, vgl. DE-C 30 19 632.

2

Die vorliegende Erfindung beruht auf der Erkenntnis, daß sich hydrophobierte Hydrotalcite für die Ethoxylierung bzw. Propoxylierung von Verbindungen mit aktiven H-Atomen sowie von Fettsäureestern eignen; diese Erkenntnis ist überraschend, denn unbehandelte natürliche bzw. synthetische Hydrotalcite, d.h. solche in nicht-calcinierter Form, sind als Ethoxylierungs- bzw. Propoxylierungskatalysatoren nicht aktiv.

Unter Verbindungen mit aktiven H-Atomen im Sinne der Erfindung sind z.B. Fettalkohole, Fettsäuren und Amine zu verstehen, die bei der Ethoxylierung bzw. Propoxylierung nichtionische Detergentien bilden. Ein typisches Beispiel hierfür ist die Umsetzung von Fettalkoholen mit üblicherweise 10 bis 18 Kohlenstoffatomen mit Ethylenoxid und/oder Propylenoxid in Gegenwart von Katalysatoren, wobei die Fettalkohole mit mehreren Molekülen Ethylenoxid und/oder Propylenoxid reagieren.

Als Katalysatoren für die vorgenannte Polyalkoxylierung sind hierfür u.a. die folgenden eingesetzt worden:

Calcium- und Strontiumhydroxide, -alkoxide und phenoxide (EP-A 00 92 256),

Calciumalkoxide (EP-A 00 91 146),

Bariumhydroxid (EP-B 0 115 083),

basische Magnesiumverbindungen, z.B. Alkoxide (EP-A 00 82 569),

Magnesium- und Calciumfettsäuresalze (EP-A 0 85 167).

Die vorgenannten Katalysatoren weisen u.a. den Nachteil auf, daß sie schlecht in das Reaktionssystem einarbeitbar und/oder schwierig herstellbar sind.

Gebräuchliche Polyalkoxylierungskatalysatoren sind weiterhin Kaliumhydroxid und Natriummethylat.

Für Fettalkoholpolyalkoxylate ist eine enge Bandbreite des Polyalkoxylierungsgrades von besonderer Bedeutung, vgl. JAOCS, Vol. 63, 691 - 695 (1986), und HAPPI, 52 - 54 (1986). Die sogenannten "narrow-range"-Alkoxylate weisen demnach insbesondere die folgenden Vorteile auf:

- niedrige Fließpunkte
- höhere Rauchpunkte
- weniger Mole Alkoxid zum Erreichen der Wasserlöslichkeit
- weniger Hydrotope für das Einbringen in flüssige Universalwaschmittel
- ein geringerer, durch Anwesenheit freier (nicht umgesetzter) Fettalkohole bedingter Geruch
- Reduzierung des Plumings beim Sprühtrocknen von Waschmittelslurries, die Fettalkoholpolyalkoxylat-Tenside enthalten.

Unter erfindungsgemäßer Verwendung hydrophobierter Hydrotalcite als Katalysatoren können Verbindungen mit aktiven H-Atomen und Fettsäureester bei kurzen Reaktionszeiten mit hohen Ausbeuten polyalkoxyliert werden; dabei weisen die Reaktionsprodukte - ebenso wie die mit calcinierten Hydrotalciten erhaltenen - eine enge Bandbreite bzw. Homologenverteilung auf, wobei die Verteilungskurve der nach Poisson berechneten sehr nahe kommt. Die erfindungsgemäß eingesetzten hydrophobierten Hydrotalcite weisen den Vorteil auf, daß sie in das Reaktionsgemisch der Alkoxylierung leicht eingearbeitet werden und wegen ihrer Unlöslichkeit in dem Reaktionsgemisch durch einfache Maßnahmen wieder abgetrennt werden können. Sie können jedoch auch in dem Reaktionsgemisch verbleiben, wenn ihre Anwesenheit bei der Weiterverwendung der Reaktionsprodukte nicht stört.

Beispiele für erfindungsgemäß unter Verwendung von hydrophobierten Hydrotalciten alkoxylierbare Verbindungen sind im folgenden aufgeführt.

Fettsäuren:

Fettsäuren mit 8 bis 22 Kohlenstoffatomen natürlicher oder synthetischer Herkunft, insbesondere geradkettige, gesättigte oder ungesättigte Fettsäuren einschließlich technischer Gemische derselben, wie sie durch Fettspaltung aus tierischen und/oder pflanzlichen Fetten und Ölen zugänglich sind, z.B. aus Kokosöl, Palmkernöl, Palmöl, Soyaöl, Sonnenblumenöl, Rüböl, Baumwollsaatöl, Fischöl, Rindertalg und Schweineschmalz; spezielle Beispiele sind Capryl-, Caprin-, Laurin-, Laurolein-, Myristin-, Myristolein-, Palmitin-, Palmitolein-, Öl-, Elaidin-, Arachin-, Gadolein-, Behen-, Brassidin- und Erucasäure; weiterhin methylverzweigte, gesättigte und ungesättigte Fettsäuren mit 10 bis 22 Kohlenstoffatomen, die bei der Dimerisierung von den entsprechenden ungesättigten Fettsäuren als Nebenprodukte entstehen, und Monocarbonsäuren mit 1 bis 7 Kohlenstoffatomen.

Hydroxyfettsäuren:

Natürliche oder synthetische Hydroxyfettsäuren, insbesondere mit 16 bis 22 Kohlenstoffatomen, z.B. Ricinolsäure oder 12-Hydroxystearinsäure.

Fettsäureamide:

Derivate der oben genannten geradkettigen, gesättigten oder ungesättigten Fettsäuren mit Ammoniak oder primären aliphatischen Aminen mit 1 bis 4 Kohlenstoffatomen in dem aliphatischen Substituenten.

Alkanole:

Gesättigte oder ungesättigte Monoalkanole, insbesondere Hydrierungsprodukte der oben genannten geradkettigen, gesättigten oder ungesättigten Fettsäuren bzw. Derivate derselben wie Methylester oder Glyceride; aliphatische oder cyclische Alkanole mit 2 bis 6 Kohlenstoffatomen, z.B. Ethanol, Propanol, Butanol, Hexanol und Cyclohexanol; einschließlich der von den vorgenannten Monoalkanolen abgeleiteten Guerbet-Alkohole.

Alkylphenole:

Mono-, Di- oder Trialkylphenole, insbesondere mit 4 bis 12 Kohlenstoffatomen in den Alkylgruppen.

Polyglykole:

Polyethylen- oder Polypropylenglykole (durchschnittlicher Polymerisationsgrad 2 bis 2000).

Fettamine:

Insbesondere primäre Fettamine, die aus Nitrilen der oben genannten geradkettigen, gesättigten oder ungesättigten Fettsäuren oder den entsprechenden Fettalkoholen zugänglich sind; weiterhin auch Mono- und Dialkylamine mit $C_1$-$C_6$-Alkylgruppen.

Fettsäurealkanolamide:

Derivate der oben genannten geradkettigen, gesättigten oder ungesättigten Fettsäuren mit Mono- oder Dialkanolaminen, insbesondere Mono- oder Diethanolamin.

Vicinal hydroxy, alkoxy-substituierte Alkane:

Ringöffnungsprodukte von 1,2-Epoxyalkangemischen mit 12 bis 22 Kohlenstoffatomen in der Kette mit mehrwertigen Alkanolen mit 2 bis 12 Kohlenstoffatomen und 2 bis 6 Hydroxylgruppen; diese Verbindungen jedoch nur, wenn sie mit Ethylenoxid oder zunächst mit Ethylenoxid und anschließend mit Propylenoxid umgesetzt werden.

Fettsäureester:

Ester, gebildet von den gegebenenfalls methylverzweigten Fettsäuren bzw. Monocarbonsäuren und Hydroxyfettsäuren gemäß der obigen Aufstellung und den Alkanolen der obigen Aufstellung; weiterhin Ester dieser Säuren mit Polyolen, z.B. mit Ethylenglykol, 1,2-Propylenglykol, 1,2-Butylenglykol, Neopentylglykol, Glycerin, Diglycerin, Triglycerin, Tetraglycerin, Trimethylolpropan, Di-trimethylolpropan, Pentaerythrit, Di-Pentaerythrit, und Zuckeralkohole, insbesondere Sorbitan.

Wie bereits eingangs ausgeführt wurde, können im Falle von Estern der obengenannten Fettsäuren mit den vorgenannten Polyolen diese auch als Partialester bzw. Partialester-enthaltende technische Estergemische, insbesondere in Form von Glyceriden, vorliegen.

Bevorzugte Fettsäureester für die erfindungsgemäße Ethoxylierung und/oder Propoxylierung sind von gesättigten oder ungesättigten, gegebenenfalls methylverzweigten oder gegebenenfalls hydroxysubstituierten Fettsäuren mit 8 bis 22 Kohlenstoffatomen mit Alkanolen mit 1 bis 4 Kohlenstoffatomen oder mit Glycerin gebildet.

Die Struktur der erfindungsgemäß erhaltenen ethoxylierten bzw. propoxylierten Fettsäureester ist nicht immer eindeutig feststellbar. Während Ester aus Fettsäuren und Monoalkanolen bzw. Vollester derselben mit Polyolen unter Einschub von Ethylenoxy- und/oder Propylenoxy-Einheiten in die Esterbindung reagieren dürften, läßt sich nicht feststellen, zu welchen Reaktionsprodukten die Reaktion von Ethylenoxid und/oder Propylenoxid mit Partialestern von Fettsäuren und Polyolen oder von hydroxy-substituierten Fettsäuren und Monoalkanolen führt; hier sind auch Reaktionen an den freien OH-Gruppen denkbar, und zwar insbesondere bei freien, primären OH-Gruppen.

Die erfindungsgemäß unter Verwendung von hydrophobierten Hydrotalciten herzustellenden Derivate sind handelsübliche Produkte, so daß sich eine nähere Erläuterung erübrigt. Sie werden durchweg durch Ethoxylierung und/oder Propoxylierung aktive Wasserstoffatome aufweisender Ausgangsverbindungen bzw. von Fettsäureestern hergestellt. Typische Vertreter sind beispielsweise ein Anlagerungsprodukt von 9 Mol Ethylenoxid an Kokosölfettsäure, ein Anlagerungsprodukt von 2 Mol Ethylenoxid an ein Fettalkoholgemisch der Kettenlänge C12-14, ein Anlagerungsprodukt von 3 Mol Ethylenoxid und 8 Mol Propylenoxid an ein Fettalkoholgemisch der Kettenlänge C12-18, ein Anlagerungsprodukt von 10 Mol Ethylenoxid an Nonylphenol, ein Anlagerungsprodukt von 7,3 Mol Ethylenoxid an Glycerin, ein Anlagerungsprodukt von 10 Mol Ethylenoxid an ein Diolgemisch, das durch Umsetzung eines 1,2-Epoxyalkangemisches der Kettenlänge C12-16 mit Ethylenglykol erhalten wurde, ein Anlagerungsprodukt von 12 Mol Ethylenoxid an ein Fettamingemisch der Kettenlänge C10-18 und ein Anlagerungsprodukt von 4 Mol Ethylenoxid an Kokosfettsäuremonoethanolamid; weiterhin Anlagerungsprodukte von 41 mol Ethylenoxid an Ricinusöl, Anlagerungsprodukte von 25 mol Ethylenoxid an gehärtetes Ricinusöl, Anlagerungsprodukte von 7 Gew.-Teilen Ethylenoxid an 10 Gew.-Teile eines Palmitinsäure-/Stearinsäuremono-/diglyceridgemisches mit einem Anteil von 40 bis 45 Gew.-% Monoglycerid und Anlagerungsprodukte von 20 mol Ethylenoxid an Sorbitanmonostearat.

Gemäß einer bevorzugten Ausführungsform der Erfindung lassen sich die erfindungsgemäß einzusetzenden hydrophobierten Hydrotalcite durch die allgemeine Formel I beschreiben, wobei das Zahlenverhältnis von m zu n im Bereich von 92:8 bis 0:100, insbesondere von 84:16 bis 20:80, liegt, und x, y, z, m und n wie oben definiert sind und die obigen Bedingungen gelten.

Die Anionen A der Monocarbonsäuren, die für die Hydrophobierung von Hydrotalciten zum Erhalt der erfindungsgemäß zu verwendenden Katalysatoren eingesetzt werden können, sind z.B. diejenigen der Fettsäuren, die weiter oben als typische Beispiele für alkoxylierbare Verbindungen aufgeführt sind; weiterhin z.B. Essigsäure, Propionsäure sowie Capronsäure und Montansäure. Typische Beispiele für Dicarbonsäuren, die für die Hydrophobierung von Hydrotalciten geeignet sind, sind Bernsteinsäure, Maleinsäure, Fumarsäure, Adipinsäure, Pimelinsäure, Suberinsäure (Korksäure), Sebacinsäure und dergleichen; weiterhin auch sogenannte Dimerfettsäuren, die z.B. aus Öl- oder Tallölfettsäuren erhalten werden können und 36 Kohlenstoffatome aufweisen. Bevorzugt werden die Anionen A der hydrophobierten Hydrotalcite der allgemeinen Formel I von Fettsäuren mit 6 bis 22 Kohlenstoffatomen oder von Dicarbonsäuren einschließlich Dimerfettsäuren mit 8 bis 36 Kohlenstoffatomen gebildet.

Weiterhin bevorzugt sind hydrophobierte Hydrotalcite der allgemeinen Formel I, die, bezogen auf ihr Gesamtgewicht, 5 - 70, insbesondere 10 bis 55 Gew.-% der Anionen A von Fettsäuren mit 6 bis 22 Kohlenstoffatomen oder 10 bis 60, insbesondere 15 bis 50 Gew.-% der Dianionen A der Dicarbonsäuren mit 8 bis 36 Kohlenstoffatomen enthalten.

Der Wassergehalt der erfindungsgemäß einzusetzenden hydrophobierten Hydrotalcite kann - in Abhängigkeit von der Art der Herstellung und den Trocknungsbedingungen - im Bereich von 0 bis 10 liegen; bevorzugt ist ein Bereich von 0 bis 4, der sich im allgemeinen einstellt, wenn man die hydrophobierten Hydrotalcite bei Temperaturen im Bereich von 150 bis 220°C bis zur Gewichtskonstanz (ca. 2 Stunden) trocknet.

Gemäß einer weiteren vorteilhaften Ausführungsform der Erfindung sind die unter Verwendung der hydrophobierten Hydrotalcite ethoxylierbaren bzw. propoxylierbaren Verbindungen mit aktiven H-Atomen aus der von Fettsäuren, Hydroxyfettsäuren, Fettsäureamiden, Alkanolen, Alkylphenolen, Polyglykolen, Fettaminen, Fettsäurealkanolamiden oder vicinal hydroxy, alkoxy-substituierten Alkanolen gebildeten Gruppe ausgewählt sind.

Weiterhin bevorzugt ist, die hydrophobierten Hydrotalcite in einer Menge von 0,1 bis 3 Gew.-%, bezogen auf das Endprodukt der Ethoxylierung bzw. Propoxylierung, einzusetzen.

Die Herstellung der erfindungsgemäß einzusetzenden hydrophobierten Hydrotalcite kann auf verschiedenen Wegen erfolgen, z.B. durch Umsetzung von natürlichen oder synthetischen Hydrotalciten in Anwesenheit oder in Abwesenheit von Lösemitteln mit den Mono- oder Dicarbonsäuren. Die hydrophobierten Hydrotalcite können weiterhin auch durch direkte Synthese unter den Bedingungen der Herstellung von Hydrotalciten in Gegenwart der Mono- oder Dicarbonsäuren erhalten werden. Erfolgt die Herstellung unter Luft- bzw. Kohlendioxidausschluß, werden carbonatfreie hydrophobierte Hydrotalcite der obigen allgemeinen Formel (m = 0) erhalten; in Anwesenheit von Kohlendioxid werden Carbonationen in die Schichtstruktur des Hydrotalcits eingebaut (m > 0). Schließlich können die hydrophobierten Hydrotalcite auch aus calziniertem Hydrotalcit durch Umsetzung desselben mit den Mono- oder Dicarbonsäuren erhalten werden; dabei können, wie vorstehend erläutert wurde, in Abwesenheit oder in Gegenwart von Kohlendioxid carbonatfreie oder carbonathaltige Produkte erhalten werden. Eine nähere Erläuterung dieser Herstellungsmethoden erfolgt im Zusammenhang mit den Ausführungsbeispielen. Anhand von Röntgenbeugungsdiagrammen konnte gezeigt werden, daß in den hydrophobierten Hydrotalciten die Hydrotalcit-Schichtstruktur unter Aufweitung der Schichtabstände erhalten geblieben ist.

Die Erfindung wird im folgenden anhand von bevorzugten Ausführungsbeispielen näher erläutert.

Die erfindungsgemäß einzusetzenden hydrophobierten Hydrotalcite können nach den folgenden Methoden erhalten werden:

1. Herstellung in Lösemitteln.

a) 50 g Hydrotalcit (handelsübliche Qualität) wurden in 250 ml Isopropanol suspendiert und innerhalb von 30 min mit 55 g Ölsäure (Molverhältnis Hydrotalcit : Ölsäure 1:2) in 200 ml Isopropanol bei Raumtemperatur versetzt.

Während des Erhitzens auf Rückflußtemperatur setzte eine $CO_2$-Entwicklung ein. Nach beendeter Gasentwicklung ließ man noch 1 bis 2 Stunden nachreagieren, ließ abkühlen und filtrierte die Suspension. Der Filterkuchen wurde mit Isopropanol nachgewaschen und im Trockenschrank bei 105°C und 100 hPa bis zur Gewichtskonstanz getrocknet. Ausbeute 71,5 g; Ölsäuregehalt ca. 40,6 Gew.-%, bezogen auf Gesamtgewicht.

b) Analog zu der in 1a) beschriebenen Arbeitsweise wurde ein hydrophobierter Hydrotalcit erhalten, wenn man anstelle der Ölsäure 33,2 g Laurinsäure einsetzte. Ausbeute 56,4 %; Laurinsäuregehalt ca. 26,4 Gew.-%, bezogen auf Gesamtgewicht; Carbonatgehalt: 6,9 Gew.-%.

c) Analog zu der unter 1a) beschriebenen Arbeitsweise erhielt man ein mit Capronsäure hydrophobiertes Produkt, wenn man anstelle der Ölsäure 19,5 g Capronsäure einsetzte. Ausbeute 67,3 g; Capronsäuregehalt ca. 14,1 Gew.-%, bezogen auf Gesamtgewicht.

d) Die Hydrophobierung analog zu der Arbeitsweise 1a) mit Korksäure (Suberinsäure) ergab das gewünschte, hydrophobierte Produkt. Ausbeute 23,3 g; Korksäuregehalt ca. 27,4 Gew.-%, bezogen auf Gesamtgewicht.

e) Die Hydrophobierung mit einer handelsüblichen C36-Dimerfettsäure analog zu der Arbeitsweise 1a) ergab das gewünschte, hydrophobierte Produkt. Ausbeute 29,4 g; Dimerfettsäuregehalt ca. 39,2 Gew.-%, bezogen auf Gesamtgewicht.

f) Die Hydrophobierung erfolgte analog zu der Arbeitsweise 1a), jedoch in Wasser als Lösemittel anstelle von Isopropanol und bei 80°C. Ausbeute 85,1 g; Laurinsäuregehalt ca. 37,6 Gew.-%, bezogen auf Gesamtgewicht.

2. Direkte Umsetzung im Kneter (ohne Lösemittel).

a) 50 g handelsüblicher Hydrotalcit wurden mit 5 g Laurinsäure 1 h bei 80°C in einem Laborkneter verarbeitet. Nach dem Abkühlen erhielt man den hydrophobierten Hydrotalcit als pulverförmiges Produkt. Laurinsäuregehalt ca. 9,1 Gew.-%, bezogen auf Gesamtgewicht.

b) Analog zu der unter 2a) beschriebenen Arbeitsweise erhielt man mit 5 g Behensäure anstelle der Laurinsäure einen hydrophobierten, pulverförmigen Hydrotalcit. Behensäuregehalt ca. 9,0 Gew.-%, bezogen auf Gesamtgewicht.

Mit dieser Verfahrensvariante lassen sich auch hydrophobierte Hydrotalcite erhalten, die bis zu 70 Gew.-% Laurin- bzw. Behensäure enthalten.

3. Direkte Synthese der hydrophoben Hydrotalcite.

a) Konventionelle Fällung.

Zu Lösungen aus 65 g 50 %-iger Natronlauge und

I. 30,4 g Natriumoleat,

II. 22 g Natriumlaurat und

III. 13,8 g Natriumcapronat

in jeweils 400 ml Wasser wurde unter intensivem Rühren eine Lösung aus 77,0 g $Mg(NO_3)_2.6H_2O$ und 37,5 g $Al(NO_3)_3.9H_2O$ in 300 ml Wasser innerhalb von 90 min zugetropft. Es bildete sich ein farbloser Niederschlag. Die Suspension wurde bei 70°C 15 h lang gerührt. Nach dem Abfiltrieren und Waschen mit Wasser wurden die erhaltenen Niederschläge im Trockenschrank bei 105°C und 100 hPa getrocknet. Ausbeuten und Analysen:

I.: 54,2 g, Ölsäuregehalt ca. 53,5 Gew.-%; Carbonatgehalt: 1,3 Gew.-%.

II.: 45,1 g, Laurinsäuregehalt ca. 38,6 Gew.-%; Carbonatgehalt: 1,3 Gew.-%.

III.: 36,8 g, Capronsäuregehalt ca. 10,7 Gew.-%. Carbonatgehalt: 2,9 Gew.-%.

b) Flash-Fällung.

Lösungen aus jeweils 307,7 g $Mg(NO_3)_2.6H_2O$ und 150,0 g $Al(NO_3)_3.9H_2O$ in 8 l Wasser wurden mit Lösungen aus je 300 g 50 %-iger Natronlauge und

I. 136,2 g Behensäure,

II. 113,0 g Ölsäure,

III. 80,1 g Laurinsäure und

IV. 143,0 g technische Rüböl-Spaltfettsäure (handelsüblich)

in jeweils 8 l Wasser durch ein Y-Stück gepumpt und zur Reaktion gebracht. Dabei fielen farblose Suspensionen an, die abfiltriert und gewaschen wurden. Die erhaltenen hydrophobierten Hydrotalcite wurden bei 110°C und 100 hPa im Trockenschrank getrocknet.

Ausbeuten und Analysen:

I.: 256,8 g, Behensäuregehalt ca. 50,9 Gew.-%;

II.: 230,7 g, Ölsäuregehalt ca. 48,3 Gew.-%;

III.: 172,5 g, Laurinsäuregehalt ca. 33,7 Gew.-%;

IV.: 279,6 g, Spaltfettsäuregehalt ca. 49,7 Gew.-%.

4. Herstellung aus calciniertem Hydrotalcit.

Hydrotalcit wurde bei 500°C 2 h lang calciniert. Dabei trat ein Massenverlust an Wasser und Kohlendioxid von ca. 60 Gew.-% ein.

a) Umsetzung mit Natriumlaurat.

10 g des calcinierten Hydrotalcits wurden unter Luftzutritt in 100 ml Wasser suspendiert und mit einer Lösung aus 13,3 g Natriumlaurat in 100 ml Wasser bei Raumtemperatur versetzt. Nach Zugabe der gesamten Lösung wurde die Suspension 2 h auf 70°C erwärmt, anschließend abgekühlt und abfiltriert. Der Filterkuchen wurde mit Wasser solange nachgewaschen, bis das Waschwasser einen pH-Wert von 10 anzeigte. Der hydrophobierte Hydrotalcit wurde bis zur Massenkonstanz bei 110°C und 100 hPa getrocknet. Man erhielt 22,5 g eines farblosen Produktes; Laurinsäuregehalt ca. 38,2 Gew.-%; Carbonatgehalt 3,1 Gew.-%.

Die Wiederholung dieses Beispiels mit Natriumcapronat anstelle von Natriumlaurat unter Luftausschluß, d.h. unter Schutzgas ($N_2$), ergab einen capronatmodifizierten Hydrotalcit mit einem Carbonatgehalt von < 0,1 Gew.-%.

b) Umsetzung mit Laurinsäure.

20 g des vorstehend beschriebenen, calcinierten Hydrotalcits wurden in 200 ml Wasser suspendiert und mit einer Lösung aus 25 g Laurinsäure in 100 ml Isopropanol versetzt. Das Reaktionsgemisch wurde 3 h lang auf 70°C erwärmt, abgekühlt und abfiltriert. Nach dem Waschen des Filterkuchens mit Isopropanol wurde dieser bei 110°C und 100 hPa bis zu Massenkonstanz getrocknet. Man erhielt 42,3 g eines farblosen Produktes; Laurinsäuregehalt ca. 44,4 Gew.-%.

Allgemeine Herstellungsvorschrift zur Herstellung von Alkoxylaten von Verbindungen mit aktiven H-Atomen unter Verwendung der hydrophobierten Hydrotalcite gemäß der Erfindung.

Die zu alkoxylierende Verbindung wurde in einem Druckreaktor vorgelegt und mit dem hydrophobierten Hydrotalcit versetzt. Der Reaktor wurde mit Stickstoff gespült und 30 min bei einer Temperatur von 100°C evakuiert. Anschließend wurde die Temperatur auf 180°C gesteigert und die gewünschte Menge Ethylenoxid bzw. Propylenoxid bei einem Druck von 400 bis 500 kPa (4-5 bar) aufgedrückt. Nach Beendigung der Reaktion ließ man 30 min nachreagieren. Man erhielt das gewünschte Reaktionsprodukt nach dem Abfiltrieren.

Beispiel 1 bis 10.

Es wurden unter Verwendung verschiedener hydrophobierter Hydrotalcite C12-C14-Fettalkoholethoxylate durch Anlagerung von 2,5 mol bzw. 3,0 mol Ethylenoxid (EO) pro Mol eines handelsüblichen $C_{12}/C_{14}$-Fettalkoholschnitts (Ansatzgröße jeweils 300 g Fettalkohol) nach der oben beschriebenen Arbeitsvorschrift hergestellt. In der Tabelle 1 sind die jeweils eingesetzten hydrophobierten Hydrotalcite sowie die Art ihrer Herstellung beschrieben, weiterhin die bei der Ethoxylierung eingesetzte Konzentration des Katalysators in Gew.-%, bezogen auf zu erwartendes Endprodukt, die Reaktionszeit in h und die Hydroxylzahlen (OHZ) der Reaktionsprodukte im Vergleich zu den theoretisch zu erwartenden. Weiterhin wird in der Tabelle 1 auf die im Anhang befindlichen Zeichnungen verwiesen, aus denen sich die Homologenverteilungen der jeweils hergestellten Ethoxylate ergeben.

7

Tabelle 1: Herstellung von C12/C14-Fettalkoholethoxylaten mit 2,5 (OHZ=184,6) bzw. 3,0 EO (OHZ=172,6)

| Beispiel Nr. | Hydrophobierung des Kata. mit: | nach Methode: | Kata-Konz. in Gew.-% | Reaktionszeit in Std. | *OHZ ist - soll | Fig. Nr. |
|---|---|---|---|---|---|---|
| 1 | Laurinsäure | 1b | 2,0 | 0,75 | 180,0 - 184,6 | 1 |
| 2 | Laurinsäure | 1b | 0,5 | 1,0 | 185,0 - 184-6 | 2 |
| 3 | Capronsäure | 1c | 2,0 | 0,75 | 178,3 - 172,6 | 3 |
| 4 | Ölsäure | 1a | 2,0 | 1,0 | 177,0 - 172,6 | 4 |
| 5 | Behensäure | 2 | 0,5 | 4,0 | 184,7 - 184,6 | 5 |
| 6 | Laurinsäure | 1f | 0,5 | 1,75 | 175,0 - 172,6 | 6 |
| 7 | Laurinsäure | 2 | 0,5 | 2,25 | 184,0 - 184,6 | 7 |
| 8 | Laurinsäure | 3 | 0,5 | 1,8 | 174,0 - 172,6 | 8 |
| 9 | Korksäure | 1d | 0,5 | 1,0 | 175,3 - 184,6 | 9 |
| 10 | C36-Dimerfettsäure | 1e | 0,5 | 0,75 | 172,1 - 172,6 | 10 |
| 11 | Essigsäure | 4 | 0,5 | 1,0 | 169,5 - 172,6 | 11 |

* OHZ = Hydroxylzahl, EO = Ethylenoxid

Beispiele 12 bis 19.

Nach der oben genannten allgemeinen Arbeitsvorschrift wurden verschiedene Verbindungen mit aktiven H-Atomen unter erfindungsgemäßer Verwendung von mit Laurinsäure hydrophobierten Katalysatoren, hergestellt gemäß Methode 1a, ethoxyliert bzw. ethoxyliert und anschließend propoxyliert.

Aus der Tabelle 2 sind die jeweils hergestellten Alkoxylate, die Konzentration des eingesetzten Katalysators in dem Reaktionsgemisch, bezogen auf zu erwartendendes Endprodukt, die Reaktionszeit und die Kenndaten der hergestellten Alkoxylate ersichtlich. Weiterhin enthält die Tabelle 2 einen Verweis auf die im Anhang befindlichen Zeichnungen, aus denen die erzielte Homologenverteilung für einige erhaltene Alkoxylate ersichtlich ist.

Tabelle 2: Ethoxylierung bzw. Propoxylierung verschiedener H-aktiver Verbindungen mit einem mit Laurinsäure hydrophobierten, nach Methode 1a erhaltenen Hydrotalcit.

| Beispiel Nr. | Produkt: | Kata-Konz. in Gew.-% | Reaktionszeit in Std. | *Kennzahlen: (ist - soll) | Fig. Nr. |
|---|---|---|---|---|---|
| 12 | C12/C14-Fettalkohol + 6EO | 0,5 | 1,75 | OHZ: 127,0-122,4 | 12 |
| 13 | Cyclohexanol + 3EO | 0,5 | 2,2 | OHZ: 237,0-241,4 | 13 |
| 14 | Octanol + 4EO | 0,5 | 1,3 | OHZ: 184,0-183,9 | 14 |
| 15 | C12/C18-Fettalkohol + 5EO | 0,5 | 1,3 | OHZ: 120-116,9 | 15 |
| 16 | 2-Ethylhexanol + 4EO | 0,5 | 2,2 | OHZ: 184,0-183,9 | 16 |
| 17 | Laurinsäure + 6EO | 0,5 | 5,5 | VZ: 119,0-120,5 | |
| 18 | Diethylenglykomono-butylether + 8EO + 10 PO | 0,5 | 8,0 | OHZ: 63,0-51,5 | |
| 19 | Dodecylamin + 12EO | 1,5 | 1,7 | AZ: 78,7 - 82,0 | |

* OHZ = Hydroxylzahl, VZ = Verseifungszahl, AZ = Aminzahl
EO = Ethylenoxid, PO = Propylenoxid

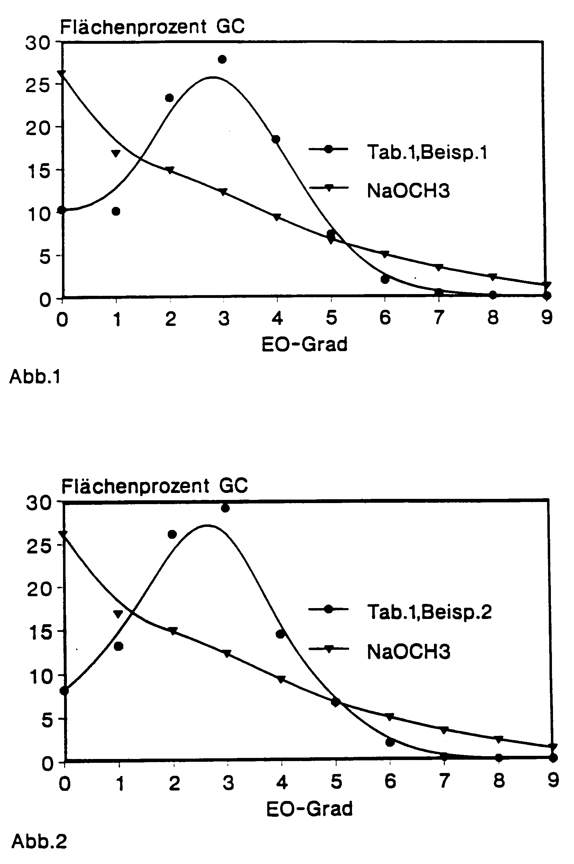

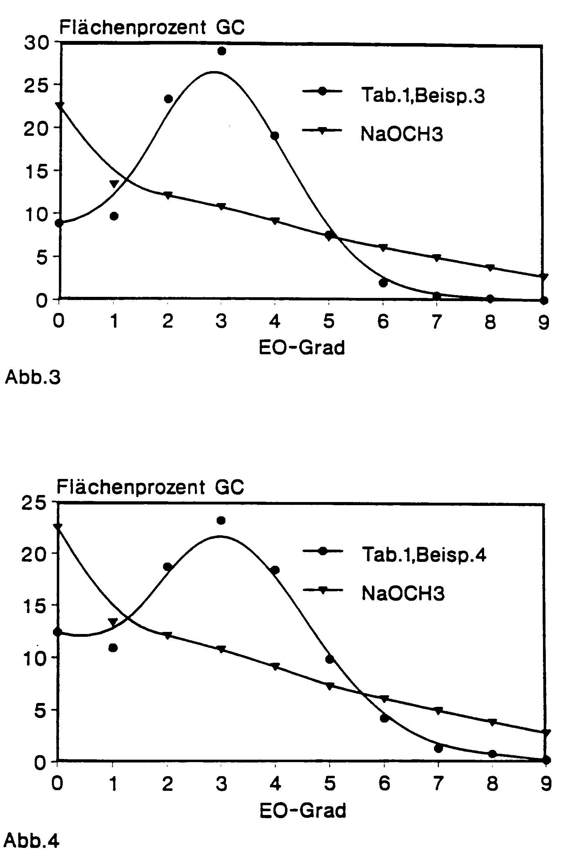

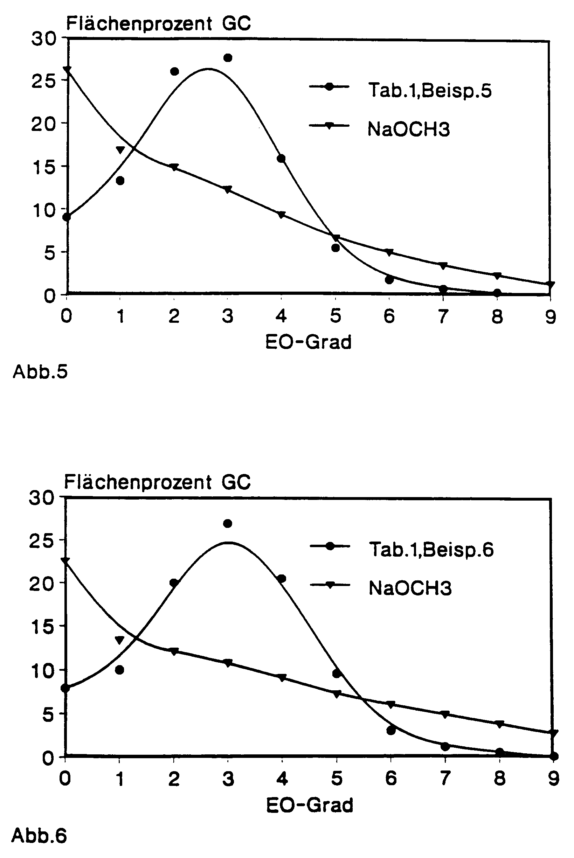

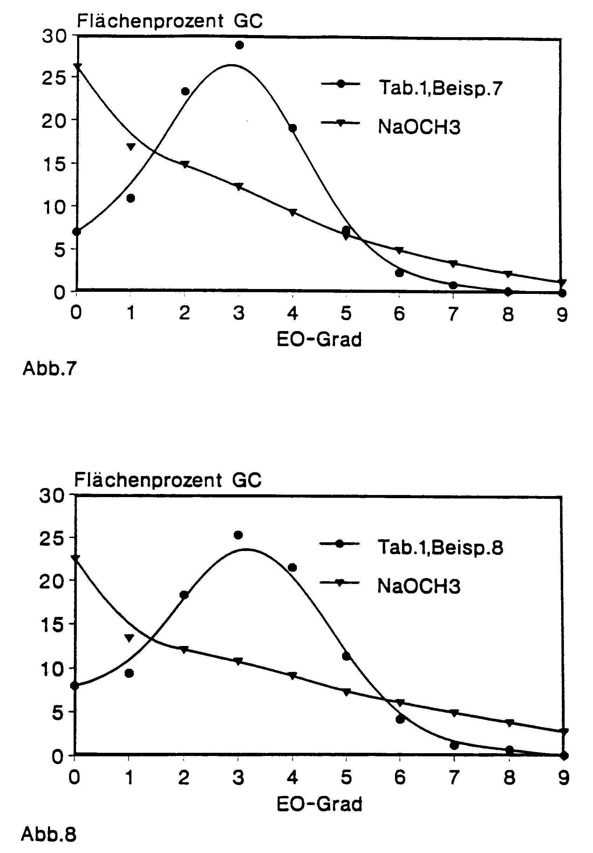

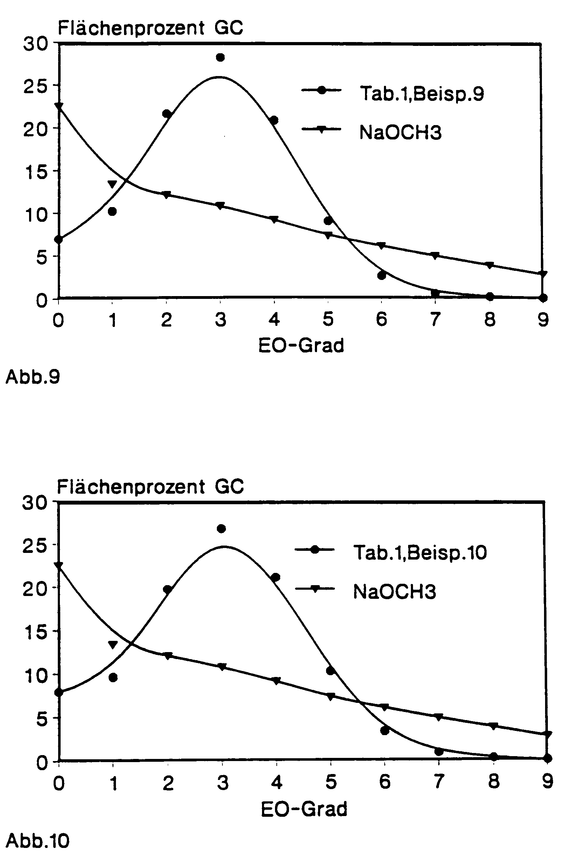

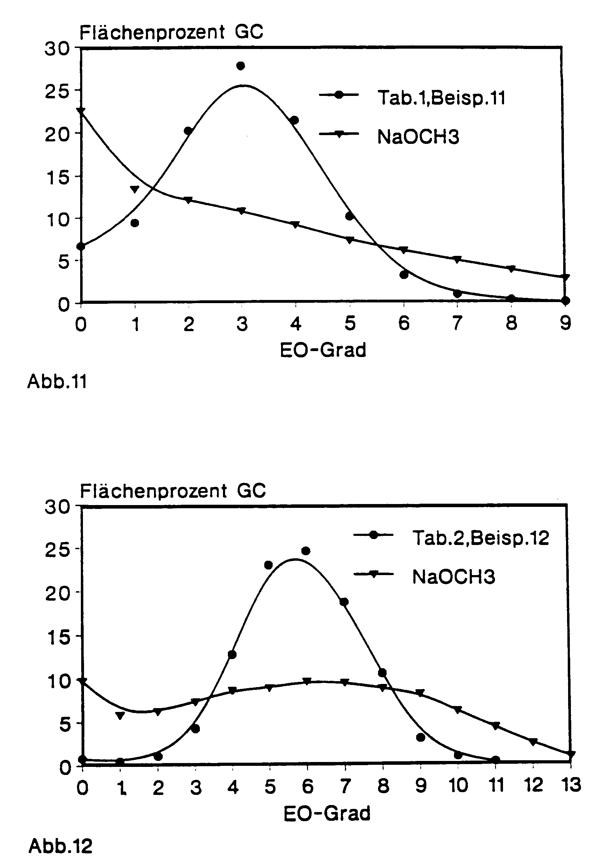

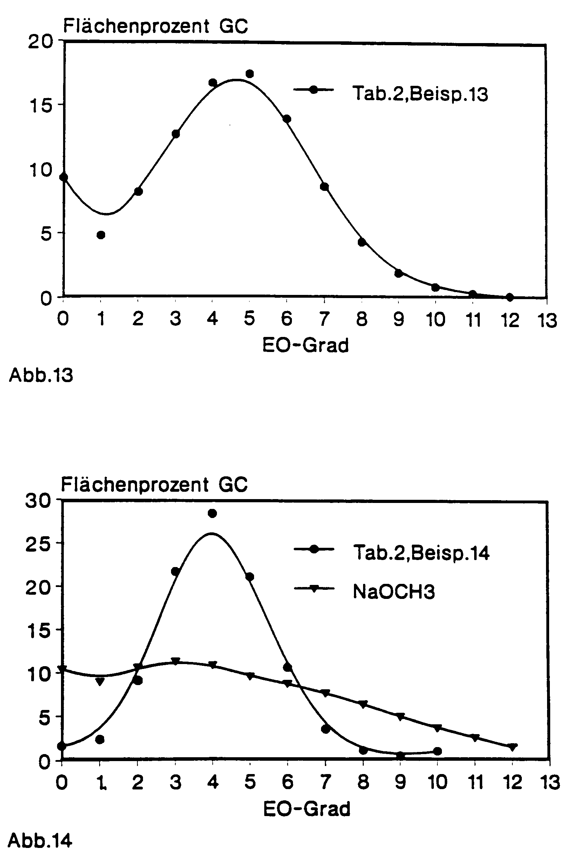

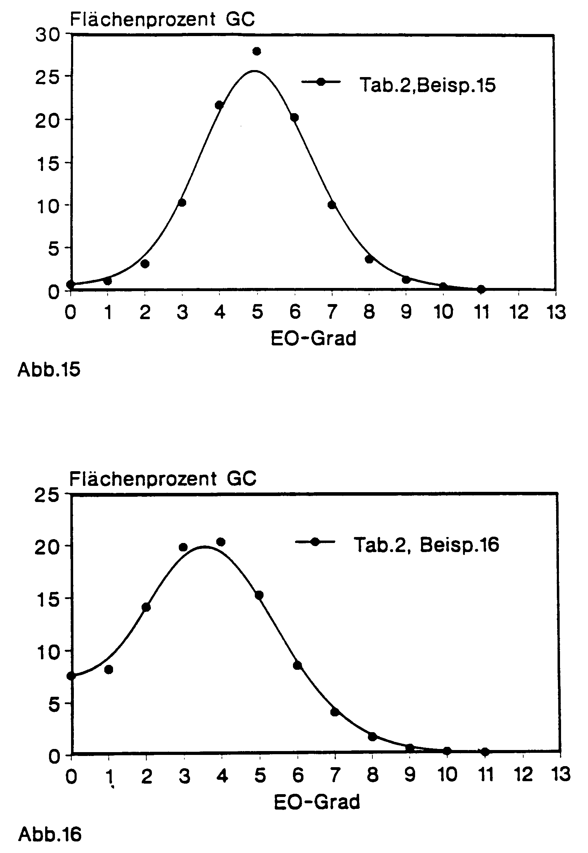

Aus den Abbildungen 1 bis 16 sind die erzielten Homologenverteilungen für die erfindungsgemäß erhaltenen Alkoxylate ersichtlich; zum Teil wurde zu Vergleichszwecken auch die Homologenverteilung für mit Natriummethylat als Ethoxylierungsskatalysator erhaltene Ethoxylate angegeben. Es zeigt sich, daß bei der erfindungsgemäßen Verwendung von hydrophobierten Hydrotalciten sehr günstige Homologenverteilungen erreicht werden.

**Patentansprüche**

1. Verwendung von hydrophobierten Hydrotalciten der allgemeinen Formel I

$$Mg_xAl(OH)_y(CO_3)_m(A)_n \cdot z\,H_2O \qquad (I)$$

in der
A das Dianion einer aliphatischen Dicarbonsäure mit 4 bis 44 Kohlenstoffatomen oder zwei Anionen von aliphatischen Monocarbonsäuren mit 2 bis 34 Kohlenstoffatomen bedeutet und die Bedingungen

$1 < x < 5,$
$y \geq 2x + 2$
$[y + 2(m + n)] = 2x + 3,$
$m + n \leq 0,5$
$m \geq 0,$
$n > 0$ und
$0 < z < 10$

gelten,
als Katalysatoren für die Ethoxylierung bzw. Propoxylierung von Verbindungen mit aktiven H-Atomen oder von Fettsäureestern, welche aus der von Estern von gegebenenfalls hydroxy-substituierten Fettsäuren mit 2 bis 22 Kohlenstoffatomen mit Monoalkanolen mit 1 bis 22 Kohlenstoffatomen sowie von Partialestern und Vollestern von gegebenenfalls hydroxy-substituierten Fettsäuren mit 2 bis 22 Kohlenstoffatomen mit Polyolen mit 2 bis 12 Kohlenstoffatomen und 2 bis 6 Hydroxylgruppen gebildeten Gruppe ausgewählt sind.

2. Verwendung nach Anspruch 1, dadurch gekennzeichnet, daß in den hydrophobierten Hydrotalciten der allgemeinen Formel I das Zahlenverhältnis von m zu n im Bereich von 92:8 bis 0:100, insbesondere von 84:16 bis 20:80 liegt, wobei x, y, z, m und n wie oben definiert sind und die obigen Bedingungen gelten.

3. Verwendung nach Anspruch 1 oder 2, dadurch gekennzeichnet, daß die Anionen A von Fettsäuren mit 6 bis 22 Kohlenstoffatomen abgeleitet sind.

4. Verwendung nach Anspruch 3, dadurch gekennzeichnet, daß die hydrophobierten Hydrotalcite, bezogen auf ihr Gesamtgewicht, 5 bis 70, insbesondere 10 bis 55 Gew.-% der Anionen A von Fettsäuren mit 6 bis 22 Kohlenstoffatomen enthalten.

5. Verwendung nach Anspruch 1 oder 2, dadurch gekennzeichnet, daß die Dianionen A von aliphatischen Dicarbonsäuren einschließlich Dimerfettsäuren mit 8 bis 36 Kohlenstoffatomen abgeleitet sind.

6. Verwendung nach Anspruch 5, dadurch gekennzeichnet, daß die hydrophobierten Hydrotalcite, bezogen auf ihr Gesamtgewicht, 10 bis 60, insbesondere 15 bis 50 Gew.-% der Dianionen A von aliphatischen Dicarbonsäuren einschließlich Dimerfettsäuren mit 8 bis 36 Kohlenstoffatomen enthalten.

7. Verwendung nach mindestens einem der Ansprüche 1 bis 6, dadurch gekennzeichnet, daß in der allgemeinen Formel I z eine Zahl im Bereich von > 0 bis 4 ist.

8. Verwendung nach mindestens einem der Ansprüche 1 bis 7, dadurch gekennzeichnet, daß die Verbindungen mit aktiven H-Atomen aus der von Fettsäuren, Hydroxyfettsäuren, Fettsäureamiden, Alkanolen, Alkylphenolen, Polyglykolen, Fettaminen, Fettsäurealkanolamiden oder vicinal hydroxy, alkoxy-substituierten Alkanolen gebildeten Gruppe ausgewählt sind.

EP 0 523 089 B1

9. Verwendung nach mindestens einem der Ansprüche 1 bis 8, dadurch gekennzeichnet, daß man die hydrophobierten Hydrotalcite in einer Menge von 0,1 bis 3 Gew.-%, bezogen auf das Endprodukt der Ethoxylierung bzw. Propoxylierung, einsetzt.

**Claims**

1. The use of hydrophobicized hydrotalcites corresponding to general formula I

$$Mg_xAl(OH)_y(CO_3)_m(A)_n \bullet z\,H_2O \qquad (I)$$

in which
A is the dianion of a aliphatic dicarboxylic acid containing 4 to 44 carbon atoms or two anions of aliphatic monocarboxylic acids containing 2 to 34 carbon atoms and the conditions

$1 < x < 5$,
$y \geq 2x + 2$
$[y + 2(m + n)] = 2x + 3$,
$m + n \leq 0.5$
$m \geq 0$,
$n > 0$ and
$0 < z < 10$

apply,
as catalysts for the ethoxylation or propoxylation of compounds containing active H atoms and of fatty acid eaters selected from the group consisting of eaters of optionally hydroxy-substituted fatty acids containing 2 to 22 carbon atoms with monoalkanols containing 1 to 22 carbon atoms and of partial eaters and full eaters of optionally hydroxy-substituted fatty acids containing 2 to 22 carbon atoms with polyols containing 2 to 12 carbon atoms and 2 to 6 hydroxyl groups.

2. The use claimed in claim 1, characterized in that, in the hydrophobicized hydrotalcites corresponding to general formula I, the ratio of m to n is in the range from 92:8 to 0:100 and, more particularly, in the range from 84:16 to 20:80, x, y, z, m and n being as defined above and the above conditions applying.

3. The use claimed in claim 1 or 2, characterized in that the anions A are derived from fatty acids containing 6 to 22 carbon atoms.

4. The use claimed in claim 3, characterized in that the hydrophobicized hydrotalcites contain 5 to 70% by weight and, more particularly, 10 to 55% by weight, based on their total weight, of the anions A of fatty acids containing 6 to 22 carbon atoms.

5. The use claimed in claim 1 or 2, characterized in that the dianions A are derived from aliphatic dicarboxylic acids, including dimer fatty acids, containing 8 to 36 carbon atoms.

6. The use claimed in claim 5, characterized in that the hydrophobicized hydrotalcites contain 10 to 60% by weight and, more particularly, 15 to 50% by weight, based on their total weight, of the dianions A of aliphatic dicarboxylic acids, including dimer fatty acids, containing 8 to 36 carbon atoms.

7. The use claimed in at least one of claims 1 to 6, characterized in that, in general formula I, z is a number in the range of > 0 to 4.

8. The use claimed in at least one of claims 1 to 7, characterized in that the compounds containing active H atoms are selected from the group consisting of fatty acids, hydroxyfatty acids, fatty acid amides, alkanols, alkylphenols, polyglycols, fatty amines, fatty acid alkanolamides or vicinally hydroxy, alkoxy-substituted alkanols.

9. The use claimed in at least one of claims 1 to 8, characterized in that the hydrophobicized hydrotalcites are used in a quantity of 0.1 to 3% by weight, based on the end product of the ethoxylation or propoxylation reaction.

11

**Revendications**

1.  Utilisation d'hydrotalcites rendues hydrophobes de formule générale I

    $Mg_xAl(OH)_y(CO_3)_m(A)_n . z H_2O$      (I)

    dans laquelle
    A signifie le dianion d'un acide dicarboxylique aliphatique ayant de 4 à 44 atomes de carbone ou deux anions d'acides monocarboxyliques aliphatiques ayant de 2 à 34 atomes de carbone et les conditions

    $1 < x < 5,$
    $y \geqq 2x + 2$
    $[y + 2(m + n)] = 2x + 3,$
    $m + n \leqq 0,5$
    $m \geqq 0,$
    $n > O$ et
    $0 < z < 10$

    sont valables,
    comme catalyseurs pour l'éthoxylation ou la propoxylation de composés ayant des atomes d'hydrogène actifs ou d'esters d'acide gras qui sont choisis dans le groupe formé des esters d'acide gras éventuellement substitués par un hydroxy ayant de 2 à 22 atomes de carbone avec des monoalcanols ayant de 1 à 22 atomes de carbone ainsi que des esters partiels et des esters complets d'acide gras éventuellement substitués par un hydroxy ayant de 2 à 22 atomes de carbone avec des polyols ayant de 2 à 12 atomes de carbone et de 2 à 6 groupes hydroxyle.

2.  Utilisation selon la revendication 1, caractérisé en ce que dans les hydrotalcites rendues hydrophobes de formule générale I, le rapport numérique de m à n se situe dans la zone de 92:8 à 0:100, en particulier de 84:16 à 20:80, x, y, z, m et n étant définis comme ci-dessus et les conditions précédentes étant valables.

3.  Utilisation selon la revendication 1 ou 2, caractérisé en ce que les anions A sont dérivés d'acides gras ayant de 6 à 22 atomes de carbone.

4.  Utilisation selon la revendication 3, caractérisée en ce que les hydrotalcites rendues hydrophobes contiennent, rapporté à leur poids total de 5 à 70 en particulier de 10 à 55 % en poids, d'anions A d'acide gras ayant de 6 à 22 atomes de carbone.

5.  Utilisation selon la revendication 1 ou 2, caractérisée en ce que les dianions A sont dérivés d'acides dicarboxyliques aliphatiques, y compris d'acides gras dimères ayant de 8 à 36 atomes de carbone.

6.  Utilisation selon la revendication 5, caractérisée en ce que les hydrotalcites rendues hydrophobes renferment de 10 à 60, en particulier de 15 à 50 % en poids rapporté à leur poids total, de dianions A d'acides dicarboxyliques aliphatiques y compris d'acides gras dimères ayant de 8 à 36 atomes de carbone.

7.  Utilisation selon au moins l'une des revendications 1 à 6, caractérisée en ce que dans la formule générale I, z est un nombre dans la zone allant de >0 à 4.

8.  Utilisation selon au moins l'une des revendications 1 à 7, caractérisée en ce que les composés ayant des atomes d'hydrogène actifs sont choisis dans le groupe formé par des acides gras, des acides gras hydroxylés, des amides d'acide gras, des alcanols, des alcylphénols, des polyglycols, des amines grasses, des alcanolamides d'acides gras et des alcanols substitués par un alcoxy et par un hydroxy vicinaux.

9.  Utilisation selon au moins une des revendications 1 à 8, caractérisée en ce que l'on met en oeuvre les hydrotalcites rendues hydrophobes en quantités allant de 0,1 à 3 % en poids, rapporté au produit final de l'éthoxylation ou de la propoxylation.

Flächenprozent GC

Abb.1

Flächenprozent GC

Abb.2

Abb.3

Abb.4

Abb.5

Abb.6

Abb.7

Abb.8

Abb.9

Abb.10

Abb.11

Abb.12

Flächenprozent GC

Abb.13

Flächenprozent GC

Abb.14

Flächenprozent GC

Abb.15

Flächenprozent GC

Abb.16